# EUROPEAN PATENT APPLICATION

(11) **EP 4 434 490 A1**
(43) Date of publication of application: **25.09.2024**
(21) Application number: 23162776.1
(22) Date of filing: 20.03.2023
(51) Int. Cl.: A61F 2/01, A61B 5/06

(54) **PERCUTANEOUS VASCULAR FILTER WITH INDUCTANCE EMBOLUS DETECTION CAPABILITY**

(71) Applicant: Koninklijke Philips N.V., 5656 AG Eindhoven (NL)
(72) Inventor: HINGSTON, John, Eindhoven (NL); MIEZWA, Megan Ann, Eindhoven (NL); CEZO, James David, 5656AG Eindhoven (NL); DELASSUS, Elodie, Eindhoven (NL); DUIGNAN, Kenneth, Eindhoven (NL)
(74) Representative: Philips Intellectual Property & Standards

(57) **Abstract**

An implantable blood filter system is provided. The implantable blood filter system includes an anchor fixedly implantable in a blood vessel of a patient; a metallic induction coil fixedly attached to the anchor; a filter fixedly attached to the induction coil or the anchor; and an external inductance measurement device. The filter includes a number of gaps having a gap size. The gap size is selected such that blood cells can pass through the gaps, and an embolus in contact with the filter cannot pass through the gaps. Thus, the embolus is captured by the filter. The external inductance measurement device is configured to detect whether an embolus has been captured by the filter.

## Description

### TECHNICAL FIELD

The subject matter described herein relates to a devices, methods, and systems for filtering and detecting emboli in a blood vessel. This implantable blood filter system has particular but not exclusive utility for long-term filtering of the carotid artery.

### BACKGROUND

Stroke is the second leading cause of death globally, and the third leading cause of severe long-term disability. Globally, approximately 14 million stroke cases are estimated to occur annually. There are estimated more than 80 million stroke survivors globally. The United States has 140,000 stroke deaths per year and 7 million stroke survivors, according to the American Stroke Association. According to US Centers for Disease Control and Prevention (CDC), about 800,000 people in the United States suffer from stoke annually, of which 80% of the cases are new.

There are two main types of stroke: ischemic stroke, which is due to the lack of blood flow, and hemorrhagic stroke, which is due to bleeding. Both cause parts of the brain to stop functioning properly. Ischemic stroke represents almost 87% of stroke cases. In the United States, nearly 200,000 of 700,000 ischemic stroke patients (about 30%) receive mechanical thrombectomy treatment. Outside of the United States, 20% of approximately 9.7 million annual cases of ischemic stroke are treated with mechanical thrombectomy.

An embolism is the lodging of an embolus (e.g., any blockage-causing piece of material) inside a blood vessel. An embolism can cause partial or total blockage of blood flow in the affected vessel. Such a blockage (e.g., a vascular occlusion) may affect a part of the body distant from the origin of the embolus. Arterial embolisms are those that follow and, if not dissolved on the way, lodge in a more distal part of the systemic circulation. Arterial embolism can cause occlusion in any part of the body. It is a major cause of infarction (e.g., tissue death from blockage of the blood supply). An embolus lodging in the brain from either the heart or a carotid artery will most likely be the cause of a stroke due to ischemia.

Preventing stroke is one of the most important clinical management goals in the treatment of atrial fibrillation. The pathogenesis of ischemic stroke in atrial fibrillation is most often an embolus emerging from the heart or large arteries and lodging in a cerebral artery. The size of the infarct and extent of brain damage are directly related to the size of the embolus. In Atrial Fibrillation patients, approximately 80% of strokes are total or partial anterior circulation strokes (major strokes) caused by occlusions of the main branches of the carotid arteries. The diameter of these branches is typically greater than 1.5 mm.

Oral anticoagulants are not enough on their own. Warfarin and non-warfarin oral anticoagulants (NOACs) reduce stroke risk by approximately 65%. However, in high-risk patients, the annual stroke risk despite taking oral anticoagulants (OACs) remains high at 2% to 4%. In addition, patients with high stroke risk, plus atrial fibrillation, who are unsuitable for OACs, require other stroke prevention strategies.

The information included in this Background section of the specification, including any references cited herein and any description or discussion thereof, is included for technical reference purposes only and is not to be regarded as subject matter by which the scope of the disclosure is to be bound.

### SUMMARY

Disclosed is an implantable blood filter system having particular, but not exclusive, utility for reducing the occurrence of ischemic stroke. The implantable blood filter system includes a (1) filter that is capable of capturing an embolus while allowing blood cells to pass through, and (2) an external measurement device, probe, or reader that is capable of detecting whether an embolus has been captured by the filter. The filter is implantable (and if necessary, replaceable) via a minimally invasive intravascular procedure. The external measurement device is configured to be usable by the patient in a home setting, with minimal effort or training. If a clot or other embolus is detected within the filter, the patient may be instructed to visit a clinician for appropriate treatment.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes an implantable blood filter system. The implantable blood filter system includes an anchor fixedly implantable in a blood vessel of a patient; a metallic induction coil fixedly attached to the anchor; a filter fixedly attached to the induction coil or the anchor; and an external inductance measurement device, where the filter includes a plurality of gaps having a gap size, where the gap size is selected such that: blood cells can pass through the plurality of gaps; and an embolus in contact with the filter cannot pass through the plurality of gaps and the embolus is captured by the filter, and where the external inductance measurement device configured to detect whether the embolus has been captured by the filter. Other embodiments of this aspect include corresponding computer systems, apparatus, and computer programs recorded on one or more computer storage devices, each configured to perform the actions of the methods.

Implementations may include one or more of the following features. In some embodiments, to detect whether the embolus has been captured by the filter, the external inductance measurement device is configured to detect an inductance signature of the embolus. In some embodiments, an inductance signature of the filter is configured to change in response to the filter being captured by the embolus, and, to detect whether the embolus has been captured by the filter, the external inductance measurement device is configured to detect the changed inductance signature. In some embodiments, the anchor includes the induction coil. In some embodiments, the anchor includes the filter. In some embodiments, the induction coil includes the filter. In some embodiments, the filter includes a plurality of filter stages. In some embodiments, the external inductance measurement device includes a handheld device. In some embodiments, the external inductance measurement device includes a wearable device. Implementations of the described techniques may include hardware, a method or process, or computer software on a computer-accessible medium.

One general aspect includes a permanently implantable blood filter. The permanently implantable blood filter includes a stent fixedly implantable in a blood vessel of a patient; an induction coil fixedly attached to the stent; and a filter fixedly attached to the stent or the induction coil, where the filter includes a coil or mesh, where the coil or mesh includes a plurality of gaps having a gap size, where the gap size is selected such that: blood cells can pass through the plurality of gaps; and an embolus in contact with the filter cannot pass through the plurality of gaps and the embolus is captured by the filter. In some embodiments, at least one of the stent or the coil or mesh includes a metal.

One general aspect includes a measurement device. The measurement device includes at least one induction coil; a user interface configured to provide visual, auditory, or haptic feedback to a user; a processor in communication with the user interface and the at least one induction coil; and a memory operatively coupled to the processor and including instructions that direct the processor to: with at least the at least one induction coil, detect whether an embolus is present in a filter device implanted within the user; and with the user interface, indicate to the user whether the embolus is present in the filter device. Other embodiments of this aspect include corresponding computer systems, apparatus, and computer programs recorded on one or more computer storage devices, each configured to perform the actions of the methods.

Implementations may include one or more of the following features. In some embodiments, the instructions further direct the processor to: with the user interface, indicate whether the measurement device is properly placed or oriented to detect the embolus in the filter device. In some embodiments, the instructions further direct the processor to: if the processor does not detect the filter device, provide, with the user interface, a first indication; or if the processor detects the filter device, provide, with the user interface, a second indication. In some embodiments, the instructions further direct the processor to: if the processor detects that the embolus is not present in the filter device, provide, with the user interface, a third indication; or if the processor detects that the embolus is present in the filter device, provide, with the user interface, a fourth indication. In some embodiments, at least one of the first indication, the second indication, the third indication, or the fourth indication includes visual, auditory, or haptic feedback. In some embodiments, the measurement device is a handheld device. In some embodiments, the measurement device is a wearable device. Implementations of the described techniques may include hardware, a method or process, or computer software on a computer-accessible medium.

One general aspect includes a method. The method includes implanting a filter device in a vessel of a patient; with a handheld or wearable measurement device, detecting whether an embolus is present in the filter device; and with a user interface, alerting the user whether the embolus is detected to be present in the filter device. In some embodiments, the method further includes, if the embolus is detected to be present in the filter device, at least one of: introducing anticoagulant medications into the patient; or removing the filter device. Implementations of the described techniques may include hardware, a method or process, or computer software on a computer-accessible medium.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the implantable blood filter system, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
**Figure 1A** is a schematic, diagrammatic, perspective view of at least a portion of an example implantable blood filter system, in accordance with at least one embodiment of the present disclosure.
**Figure 1B** is a schematic, diagrammatic, perspective view of at least a portion of an example implantable blood filter system, with the blood vessel and frame or anchor removed for clarity, in accordance with at least one embodiment of the present disclosure.
**Figure 2** is a schematic, diagrammatic, cross-sectional view of at least a portion of an example temporary implantable blood filter deployment system, in accordance with aspects of the present disclosure.
**Figure 3** is a perspective image of a temporary carotid artery filter, in accordance with aspects of the present disclosure.
**Figure 4** is a schematic, diagrammatic, side cross-sectional view of at least a portion of an example implantable blood filter system, in accordance with at least one embodiment of the present disclosure.
**Figure 5** is a schematic, diagrammatic, end cross-sectional view of at least a portion of an example implantable blood filter system, in accordance with at least one embodiment of the present disclosure.
**Figure 6** is a schematic, diagrammatic, end cross-sectional view of at least a portion of an example implantable blood filter system, in accordance with at least one embodiment of the present disclosure.
**Figure 7** is a schematic, diagrammatic, perspective view of at least a portion of an example implantable blood filter system, in accordance with at least one embodiment of the present disclosure.
**Figure 8A** is a schematic, diagrammatic, perspective view of at least a portion of an example implantable blood filter system, in accordance with at least one embodiment of the present disclosure.
**Figure 8B** is a schematic, diagrammatic, perspective view of at least a portion of an example implantable blood filter system, in accordance with at least one embodiment of the present disclosure.
**Figure 9** is a schematic, diagrammatic, perspective view of an example inductive measurement system, in accordance with at least one embodiment of the present disclosure.
**Figure 10** is a diagrammatic, schematic view of a handheld medical scanning device, according to aspects of the present disclosure.
**Figure 11** is a diagrammatic, perspective view of a user applying an external measurement device to the site of the implant to perform a self-test, in accordance with at least one embodiment of the present disclosure.
**Figure 12** is a diagrammatic, perspective view of a user wearing a wearable external measurement device, inductance probe, or reader over the site of the implant to perform a self-test, in accordance with at least one embodiment of the present disclosure.
**Figure 13** is a flow diagram showing the steps of an example implantable permanent blood filter placement method, in accordance with at least one embodiment of the present disclosure.
**Figure 14** is a schematic diagram of a processor circuit, according to embodiments of the present disclosure.

### DETAILED DESCRIPTION

In accordance with at least one embodiment of the present disclosure, an implantable blood filter system is provided that includes a permanent percutaneous carotid artery filter with embolus/clot detection capabilities. The carotid filter device is designed to be permanently implanted in the carotid artery of a patient (e.g., implanted for weeks, months, years, decades, or the lifespan of the patient), and the filter is designed such that it is straightforward to detect, using external electromagnetic inductance, when an embolus/clot has been captured.

In the current state of the art, deployable carotid artery filters (e.g., embolic protection devices) are normally placed before a carotid stenting procedure to capture any embolus/clot that may be generated by the stenting procedure. However, such embolic protection devices are temporary, and need to be removed at the end of the procedure.

There is a significant need for other stroke prevention therapies. The present disclosure outlines a novel solution with a permanent percutaneous carotid artery filter or embolic protection device designed to be easily tested using external electromagnetic inductance, to ascertain if it has captured an embolus or not. The filter has specific features in its design to facilitate the detection of a captured embolus by a low skilled operator. For example, the design is such that the patient can perform the inductance scan at home, with minimal training, to check if the filter has captured an embolus. If the filter has captured an embolus, or is suspected of having captured an embolus, the patient can then go to the local hospital to remove the embolus, e.g., with anticoagulants. However, if that fails to dissolve the embolus, the filter can be removed during a minimally invasive (e.g., intravascular) procedure and replaced with a new filter.

The permanent carotid filter is designed such that when the filter captures an embolus, the inductance signature of the filter changes in a significant and easily detectable way. The filter comes with a 'reader', that the patient can use at home to regularly check for captured embolus. For example, on a regular basis (e.g., once per week, once per day, etc., depending on risk level) the patient does a simple test at home to check if the filter captured an embolus.

The filter device may be anchored in place using a number of different state of the art options. For example, the filter may be anchored using radial force. In a manner similar to similar to traditional stents, the device may exert an outward radial force on the blood vessel that keeps the filter in place. To achieve this, the stent/filter could be a of a number of different options, including but not limited to a Nitinol braided stent, a Nitinol laser cut stent, a stainless alloy laser cut stent, stainless steel braided stent, a polymer braided stent, or a polymer laser cut stent. Instead or in addition, to prevent migration, the filter may be anchored by one or more sutures, or by any other method known in the art.

The filter part of the device has the ability to filter the blood flowing through the carotid artery, leveraging current state of art, and to have a detectably different inductance signature between its normal state (e.g., no embolus captured) and non-normal state (e.g., an embolus has been captured in the device). The filter may be or include a metallic mesh, a coil or spiral, or polymer net, mesh, coil, or any other filter type known in the art, and may include multi-stage filters as shown below. The filter may for example be designed to have a very large inductance signature. When an embolus is captured in the filter, the embolus itself will physically alter the device, resulting in a different inductance signature. This change in inductance fields may serve as a signal that the device has captured a clot or other embolus that may require the patient to obtain a professional scan.

The external test device may for example be a standard, off-the-shelf external inductance probe system, with software specific to detecting an embolus in the implanted filter and delivering a simple yes/no answer that is readily understandable by the patient. In other embodiments, the external test device may be a wearable device such as a neck wrap.

The present disclosure provides not only a permanently implantable blood filter, but also devices and methods for an individual patient to detect a captured embolus, with minimal effort and training, and thus to seek prompt medical attention to treat the embolus. As such, the implantable blood filter system may significantly reduce the incidence and/or severity of ischemic stroke (or other embolus-related ischemic injury) in the patients for whom it is deployed, and may thus improve patient outcomes. This may be accomplished at relatively low cost, as the blood filter is implantable (and if necessary, replaceable) via a minimally invasive intravascular procedure, and the testing can be performed in a home setting, without the need for clinician involvement.

The present application is related to U.S. Application No. 63/440,872, filed on January 24, 2023, hereby incorporated by reference in its entirety as though fully set forth herein. These descriptions are provided for exemplary purposes only, and should not be considered to limit the scope of the implantable blood filter system. Certain features may be added, removed, or modified without departing from the spirit of the claimed subject matter.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

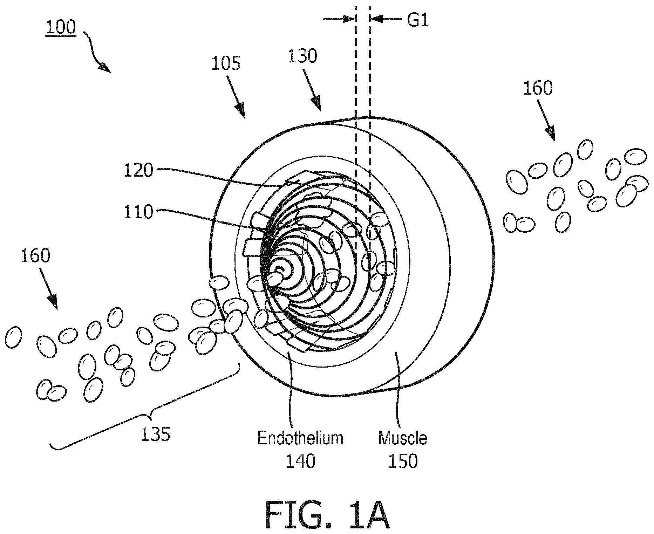

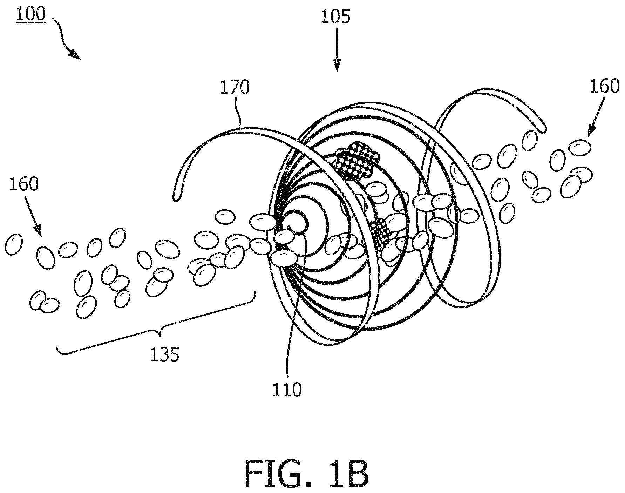

**Figure 1A** is a schematic, diagrammatic, perspective view of at least a portion of an example implantable blood filter system 100, in accordance with at least one embodiment of the present disclosure. The implantable blood filter system 100 includes an implant 105 comprising a filter 110, an anchor 120, and an inductive coil 170 (see Figure 1B), which are implantable in a blood vessel 130. The blood vessel 130 includes an endothelium or vessel wall 140 surrounded by muscle tissue 150. The blood vessel 130 carries blood 135, including blood cells 160.

In some embodiments, the filter 110 is designed to have a large inductance signature. When an embolus is captured in the filter, this will physically change the coil and therefore the inductance signature. When an embolus is captured, the change in shape of the filter can be detected by the external probe or measurement device.

The filter includes gaps of a width G1, which is large enough to allow blood cells 160 to pass through, but small enough to prevent the passage of an embolus large enough to cause an ischemic stroke, or other ischemic injury. Depending on the implementation, the frame or anchor 120 may be or include at least one of a Nitinol braided stent, a Nitinol laser cut stent, a stainless alloy laser cut stent, stainless steel braided stent. a polymer braided stent, a polymer laser cut stent, or may be or include one or more sutures.

**Figure 1B** is a schematic, diagrammatic, perspective view of at least a portion of an example implantable blood filter system 100, with the blood vessel 130 and frame or anchor 120 removed for clarity, in accordance with at least one embodiment of the present disclosure. The implantable blood filter system 100 includes an implant 105 comprising a filter 110, an anchor 120 (see Figure 1A), and an inductive coil 170. In an example, both the inductive coil 170 and the filter 110 are made from a conductive material such as a metal, the inductive coil 170 is in electrical communication with the filter 110 (and possibly the anchor 120), in such a way that an Eddy Current Testing (ECT) scan can induce the filter 110, anchor 120, and/or inductive coil 170 to produce a current and a secondary magnetic field, as described below. The filter is designed such that the inductance signature of the filter 110 changes when the filter 110 has captured an embolus.

The shape and composition of the filter 110 (e.g., a low-nickel stainless-steel coil, nitinol coil, or other biocompatible, electrically conductive coil) is selected to maximize the change in the eddy current effect when a clot has been captured. For example, the filter 110 may exhibit a strong eddy current signal when no clot has been captured, and a weaker eddy current signal when a clot has been captured. In other embodiments, the implant 105 may have a normal resonance frequency of X Hertz when no embolus has been captured, and a different (e.g., larger or smaller) normal resonance frequency of Y Hertz when an embolus has been captured, e.g., due to a change in shape of the filter 110. Thus, the change in eddy current magnitude or frequency can be used to deduce the presence of an embolus in the filter 110. In some embodiments, the frame or anchor 120 may be, may incorporate, or may be capable of acting as, an induction coil 170.

Before continuing, it should be noted that the examples described above are provided for purposes of illustration, and are not intended to be limiting. Other devices and/or device configurations may be utilized to carry out the operations described herein.

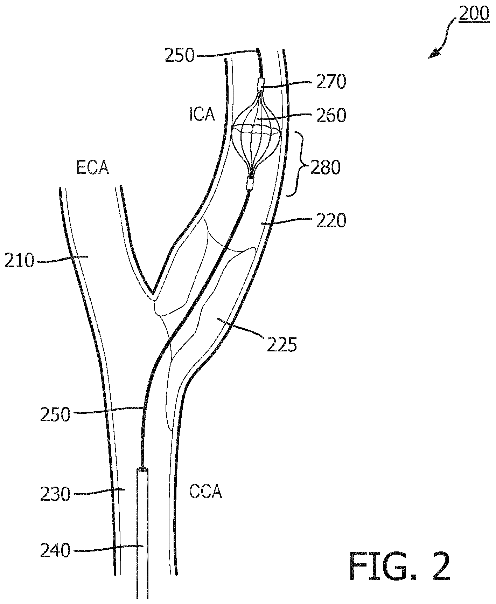

**Figure 2** is a schematic, diagrammatic, cross-sectional view of at least a portion of an example temporary implantable blood filter deployment system 200, in accordance with aspects of the present disclosure. Visible are the external carotid artery, (ECA) 210, the internal carotid artery (ICA) 220 which includes a constriction 225, the common carotid artery (CCA) 230, a catheter 240, and a guidewire 250. The temporary implantable blood filter deployment system 200 includes a temporary carotid artery filter 260 that is placed downstream (e.g., cranial) of a constriction 225 before a stenting or ablation procedure. In the example shown in Figure 2, the temporary filter 260 is incorporated directly into the guidewire 250, and can be opened and closed by a deployment mechanism 280 that, under the control of a clinician, is translated proximally and distally relative to an anchor 270.

The temporary filter 260 is placed only during the ablation or stent placement procedure, to capture material that may be dislodged during the procedure. In most cases, the temporary filter 260 must be removed after the stent is placed or the ablation is complete.

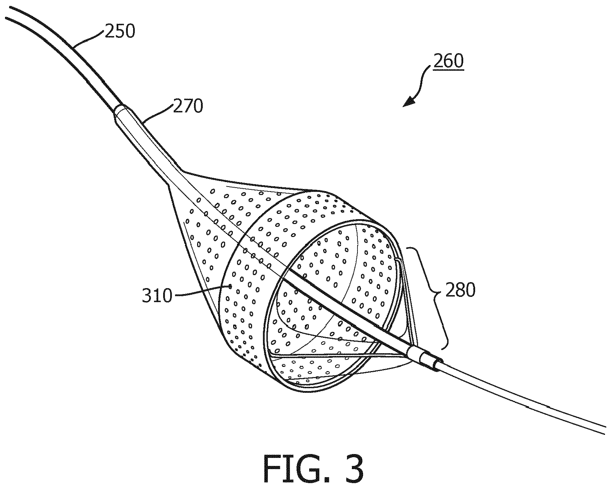

**Figure 3** is a perspective image of an example of temporary carotid artery filter 260, in accordance with aspects of the present disclosure. Visible are the guidewire 250, anchor 270, temporary filter 260, and deployment mechanism 280. The temporary filter 260 may for example be made of a polymer mesh that includes holes 310. The holes 310 are large enough to allow blood cells to pass, but small enough to capture an embolus that is capable of causing ischemic stroke.

The design of the temporary filter 260 is anchored by the catheter. It is not designed to be left in place, and has no anchoring mechanism of its own.

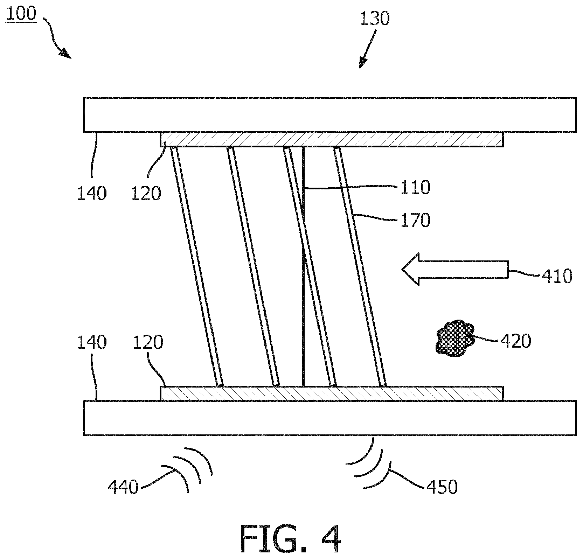

**Figure 4** is a schematic, diagrammatic, side cross-sectional view of at least a portion of an example implantable blood filter system 100, in accordance with at least one embodiment of the present disclosure. Visible are the filter 110, frame or anchor 120, and induction coil 170. The filter 110 may be co-formed with, or fixedly attached to, the frame or anchor 120 and/or the induction coil 170. The frame or anchor 120 exerts radially outward pressure on the vessel wall 140, thus preventing the filter 110 and induction coil 170 from migrating within the blood vessel 130. In the example shown in Figure 4, an embolus 420, moving in the blood flow direction 410, can be captured by the filter 110.

Also visible is alternating-current (AC) primary magnetic field 440 (e.g., emitted by an external inductance probe or "reader"), and an AC secondary magnetic field 450 emitted by the filter 110, frame 120, and/or induction coil 170. In some embodiments, detection of the embolus 420 (e.g., by an external inductance sensor) relies on the inductance signature of the embolus itself, which may return a significantly weaker secondary field 450 than the frame or anchor 120, filter 110, and induction coil 170 collectively do, or may reduce the secondary field 450 emitted by these components, or may alter the AC frequency of the secondary field 450. In other embodiments, the frame 120 and/or filter 110 may be made of high-inductance-signature materials such as metals, and an inductance probe may detect the presence of an embolus 420 by the change in shape (and thus inductance signature) of the filter 110. In each case, however, the shape and composition of the filter 110, frame 120, and inductance coil are selected such that a detectable change in inductance signature (e.g., in the intensity of the secondary magnetic field 450) occurs when an embolus 420 is captured by the filter 110.

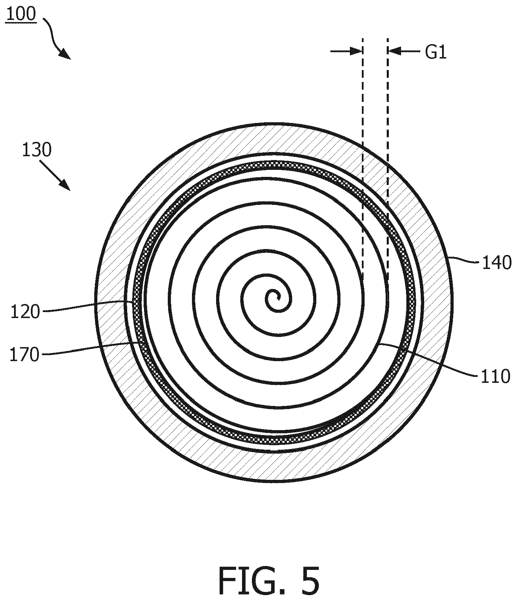

**Figure 5** is a schematic, diagrammatic, end cross-sectional view of at least a portion of an example implantable blood filter system 100, in accordance with at least one embodiment of the present disclosure. Visible are the vessel wall 140 of the blood vessel 130, along with the frame or anchor 120, the filter 110, and the inductive coil 170. In the example shown in Figure 5, the filter 110 is or includes a spiral of a material such as a metal. In other embodiments, the filter 110 may be or include a mesh, grid, perforated sheet, or other related structure that includes gaps of a width G1, which is large enough to allow blood cells to pass through, but small enough to prevent the passage of an embolus large enough to cause an ischemic stroke, or other ischemic injury.

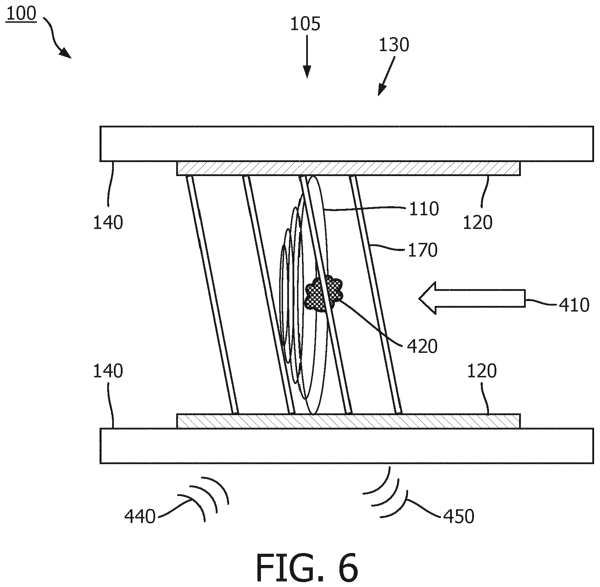

**Figure 6** is a schematic, diagrammatic, end cross-sectional view of at least a portion of an example implantable blood filter system 100, in accordance with at least one embodiment of the present disclosure. Visible are the vessel wall 140 of the blood vessel 130, the frame or anchor 120, the inductive coil 170, the filter 110, primary magnetic field 440, and secondary magnetic field 450. The arrangement of Figure 6 is similar to that shown in Figures 4 and 5, except that the embolus 420, moving in the blood flow direction 410, has been captured by the filter 110, resulting in a deformation of the filter 110 such that the inductance signature of the filter (and/or the combined inductance signature of the implant 105, comprising the frame or anchor 120, inductive coil 170, and filter 110) is altered. The resulting change in the inductance signature of the implant 105 (e.g., a change in the intensity of a secondary magnetic field induced by inductive excitation) can be detected by an external inductance probe or reader. In an embodiment the embolus 420, moving in the blood flow direction 410, has been captured by the filter 110, resulting in a deformation of the filter 110 such that the filter and/or the inductive coil are no longer in their original, which results in resulting change in the inductance signature of the implant 105. The effect of capturing the embolus or emboli may also restrict the flow through the filter, thereby potentially changing the flow pattern through the filter and resistance to flow of the filter, resulting in further deformation of the filter, which can enhance the change in the inductance signature of the implant 105.

In other embodiments, the captured embolus 420 is detected by the inductance signature of the embolus 420 itself, or by the inductance signal of a high-inductance-signature filter 110, coil, and/or frame or anchor 120 being partially blocked by the embolus 420.

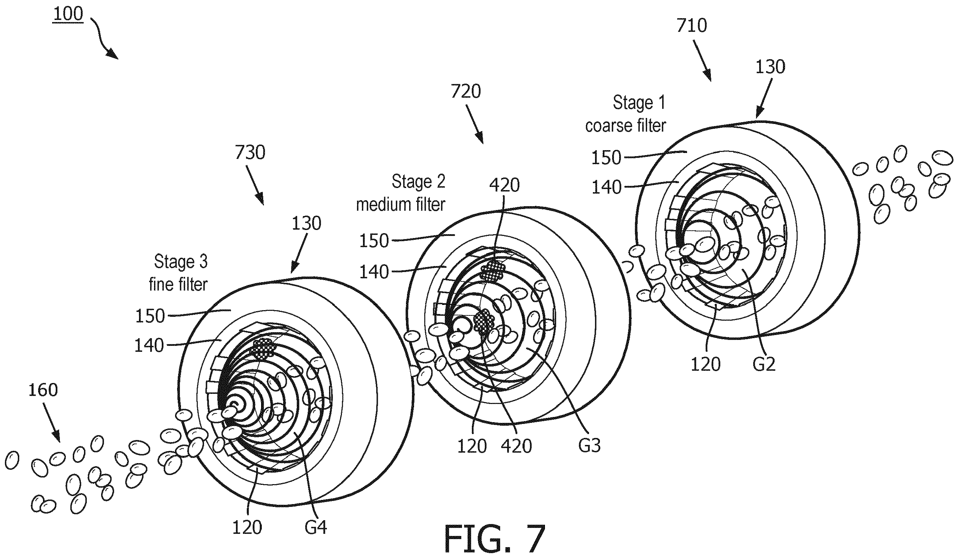

**Figure 7** is a schematic, diagrammatic, perspective view of at least a portion of an example implantable blood filter system 100, in accordance with at least one embodiment of the present disclosure. In the example shown in Figure 7, the implantable blood filter system 100 includes three frames or anchors 120 implanted within the blood vessel 130 through an outward radial force exerted on the vessel wall 140 and muscle tissue 150. In the example shown in Figure 7, the implantable blood filter system 100 includes three filters: a first filter or coarse filter 710, a second filter or medium filter 720, and a third filter or fine filter 730. In an example, the first filter or coarse filter 710 includes gaps G2 that are small enough to trap a large embolus 420, but large enough to let blood cells 160 and smaller emboli 420 pass through. Similarly, the second filter or medium filter includes gaps G3 that are small enough to trap emboli 420 of moderate or typical size, but large enough to let blood cells 160 and small emboli 420 pass through, while the third filter or fine filter includes gaps G4 that are small enough to trap small emboli 420, but large enough to let blood cells 160 pass through.

The use of three filters is merely an illustrative example that does not limit the scope of the present disclosure. Other numbers of filters may be used instead or in addition, including two, four, five, or more filters, or continuous filters. The advantage of a multi-stage filter is that it reduces the resistance of blood flow past the filter. Or put another way, it reduces the pressure differential before and after the filter. With multistage filters or continuous filters, the filter mechanism can be spread out over a length. For example, the stage 1 filter could have a most radial (outside edge) focus, while the stage 2 filter has a midsection focus, and stage 3 is focused on the center section of the blood vessel.

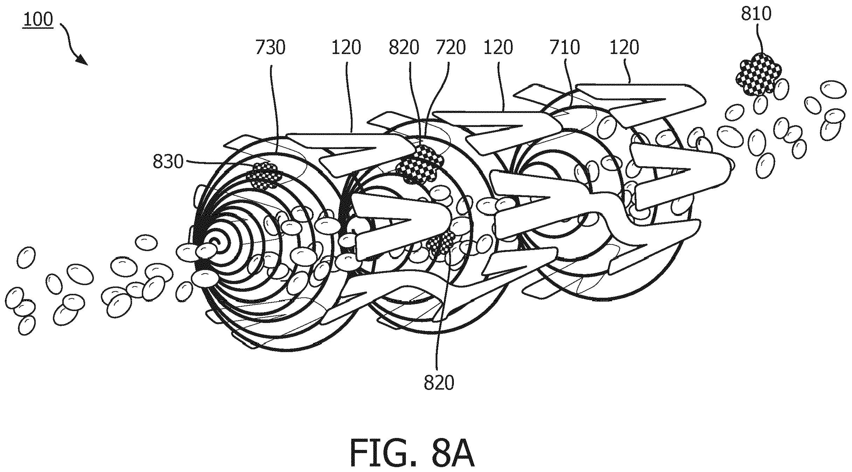

**Figure 8A** is a schematic, diagrammatic, perspective view of at least a portion of an example implantable blood filter system 100, in accordance with at least one embodiment of the present disclosure. The view of Figure 8A is similar to Figure 7, with the inductive coil 170, the vessel wall 140, and the muscle tissue 150 of the blood vessel deleted for clarity. Visible are the filters 710, 720, and 720, the frames 120, a large embolus 810, two medium-sized emboli 820, and a small embolus 830. The large embolus 810 is large enough to be captured by the large filter 810. The medium-sized emboli 820 are small enough to pass through the coarse filter 710, but large enough to be caught by the medium filter 720. The small embolus 830 is small enough to pass through the large filter 710 and medium filter 720, but large enough to be captured by the fine filter 730. In an example, emboli that are small enough to pass through the fine filter 730 may not be considered clinically significant. Although each filter or filter stage 710, 720, 730 is shown with its own anchor 120, it is noted that in some embodiments, two or more filters or filter stages may be held in place by a single anchor 120, or two or more filter stages may be connected by parts of at least one anchor.

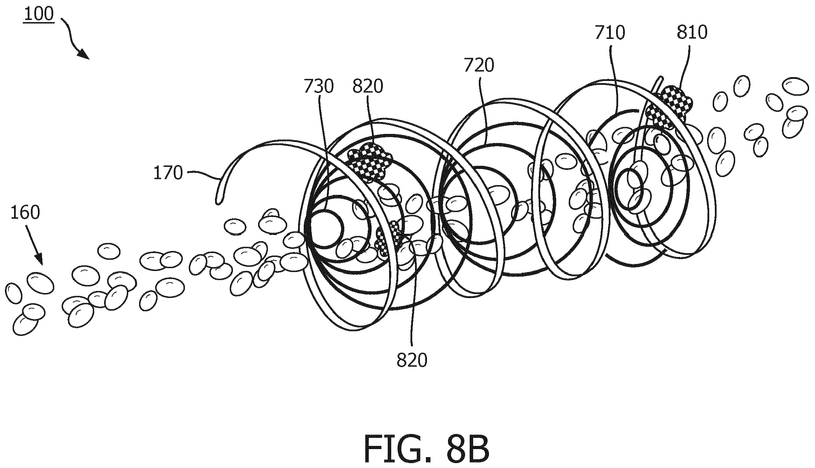

**Figure 8B** is a schematic, diagrammatic, perspective view of at least a portion of an example implantable blood filter system 100, in accordance with at least one embodiment of the present disclosure. The view of Figure 8B is the same as Figures 7 and 8A, with the frames or anchors 120, the vessel wall 140, and the muscle tissue 150 of the blood vessel deleted for clarity. Visible are the filters 710, 720, and 730, the inductive coil 170, the blood cells 160, the large embolus 810, medium emboli 820, and small embolus 830. In the example shown in Figure 8B, all three filters 710, 720, and 730 share a single inductive coil 170. However, it is noted that in some embodiments, each filter may have its own inductive coil 170, or an inductive coil 170 may be shared between two, four, or more filter stages. In some embodiments, the inductive coil 170 also serves as a frame or anchor 120. In some embodiments, the inductive coil 170 also serves as a filter. In some embodiments, the inductive coil, frame or anchor, and filter comprise a single (e.g., stent-like) object. In some embodiments, the inductive coil, frame or anchor, and filter comprise different objects that are fixedly attached (e.g., welded, soldered, brazed, etc.) to one another, or held together by any combination of tension, compression, or friction.

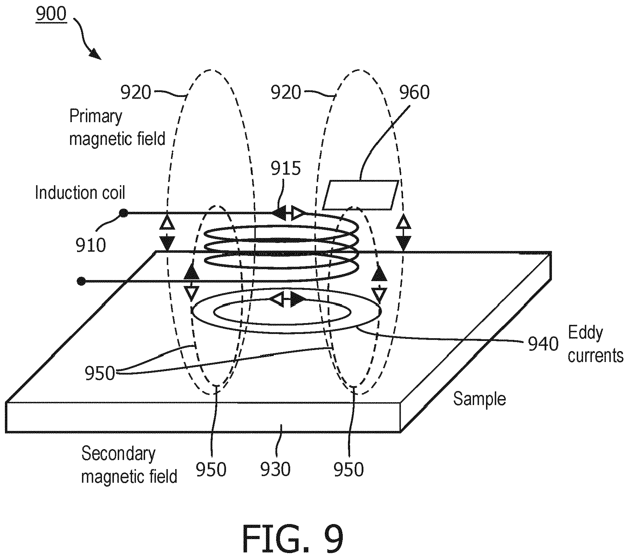

**Figure 9** is a schematic, diagrammatic, perspective view of an example inductive measurement system 900, in accordance with at least one embodiment of the present disclosure. Visible is an induction coil 910 that produces a primary magnetic field 920 when a current 915 is passed through it. In cases where the current 915 is constant (e.g., direct current or DC), the primary magnetic field 920 may be time-invariant. However, in cases where the current 915 varies periodically over time (e.g., alternating current or AC), the primary magnetic field may be time-variant. When the induction coil 910 is brought into proximity with an electrically conductive sample 930, eddy currents 940 may be induced in the sample 930, giving rise to a secondary magnetic field 950, which may for example be constant or time-variant, depending on the primary induction coil input.

Eddy currents 940 (also called Foucault's currents) are loops of electrical current induced within conductors by a changing magnetic field in the conductor according to Faraday's law of induction. Eddy currents 940 flow in closed loops within conductors, in planes perpendicular to the magnetic field 920. They can be induced within nearby stationary conductors by a time-varying magnetic field 920 created by an AC electromagnet or transformer, for example, or by relative motion between a magnet and a nearby conductive material sample 930. The magnitude of the current 940 in a given loop is proportional to the strength of the magnetic field 920, the area of the loop, and the rate of change of flux, and inversely proportional to the resistivity of the material sample 930. When graphed, these circular currents within a piece of metal can look similar to eddies or whirlpools in a liquid.

From Lenz's law, an eddy current 940 creates a magnetic field 950 that opposes the change in the magnetic field that created it, and thus eddy currents react back on the source of the magnetic field (e.g., the induction coil 910). For example, a nearby conductive surface will exert a drag force on a moving magnet that opposes its motion, due to eddy currents induced in the surface by the moving magnetic field. This effect is employed in eddy current brakes, which are used to stop rotating power tools quickly when they are turned off. The current flowing through the resistance of the conductor also dissipates energy as heat in the material. Thus, eddy currents are a cause of energy loss in alternating current (AC) inductors, transformers, electric motors and generators, and other AC machinery, requiring special construction such as laminated magnetic cores or ferrite cores to minimize the occurrence of eddy currents. Eddy currents are also used to detect cracks and flaws in metal parts using eddy-current testing systems 900.

Eddy-Current Testing (ECT) is an electromagnetic testing method that can be used in non-destructive testing (NDT), making use of electromagnetic induction to detect and characterize surface and sub-surface flaws in conductive materials. Eddy current testing can be an inspection technique for flaw detection, as well as thickness and conductivity measurements.

In its most basic form - the single-element ECT probe - a coil 910 of conductive wire is excited with an alternating electrical current 915. This wire coil produces an alternating magnetic field 920 around itself. The magnetic field 920 oscillates at the same frequency as the current 915 running through the coil 910. When the coil 910 approaches a sample 930 of conductive material, currents opposite to the ones in the coil are induced in the material - eddy currents 940.

Variations in the electrical conductivity and magnetic permeability of the test sample 930 (e.g., due to the presence of defects) causes a change in eddy current 940 and a corresponding change in phase and amplitude that can be detected by measuring the impedance changes in the coil 910, which is a telltale sign of the presence of defects. This is the basis of standard (pancake coil) ECT. NDT kits can be used in the eddy current testing process. ECT has a very wide range of applications. Since ECT is electrical in nature, it is limited to testing of conductive materials.

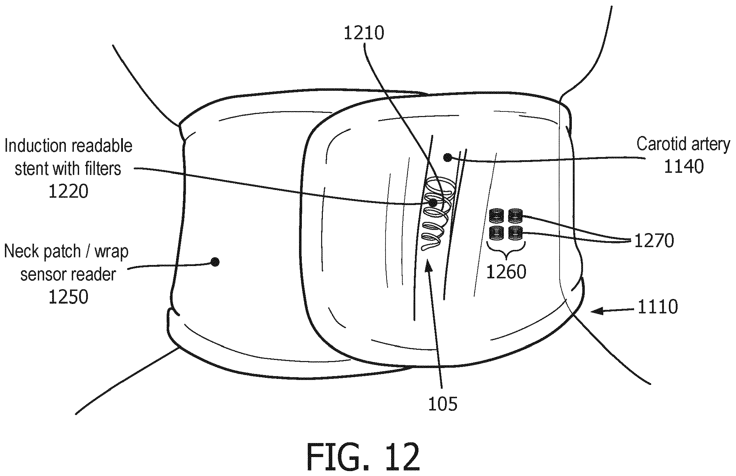

Eddy current array (ECA) and ECT share the same basic working principles. ECA technology provides the ability to electronically drive an array of coils (e.g., multiple coils 910) arranged in specific pattern (see Figure 12) called a topology that generates a sensitivity profile suited to the target defects. Data acquisition is achieved by multiplexing the coils in a special pattern to avoid mutual inductance between the individual coils. The benefits of ECA include, but are not limited to: faster inspections, wider coverage, better detection capabilities, easier analysis because of simpler scan patterns, improved positioning and sizing because of encoded data, and less dependence on the skill of the operator, since array probes yield more consistent results compared to manual raster scans. In addition, ECA probes can easily be designed to be flexible or shaped to specifications, making hard-to-reach areas easier to inspect, as shown below in Figure 12.

In some cases, depending on the implementation, detection or measurement of the secondary magnetic field 950 and/or eddy currents 940 may be accomplished with a separate field sensor 960.

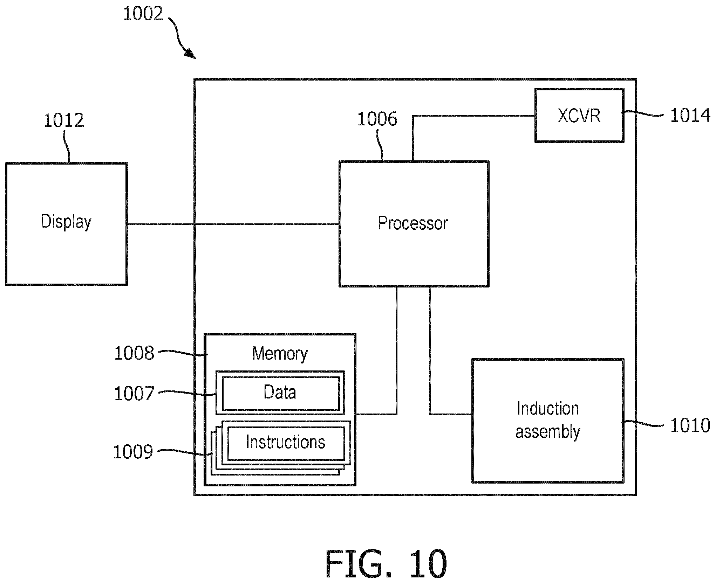

**Figure 10** is a diagrammatic, schematic view of a handheld medical scanning device 1002, according to aspects of the present disclosure. The filter comes with a 'reader', that the patient can use at home to regularly check for captured embolus. For example, once a week the patient does a simple test at home to check if the filter captured an embolus (via measurement of change in inductance).

The handheld medical scanning device 1002 includes an induction assembly 1010. The induction assembly may include a single induction coil, a 1D array of induction coils, or a 2D array of induction coils. The induction assembly emits a primary magnetic field and receives/measures a secondary magnetic field induced in the implant.

The handheld medical scanning device 1002 may include a memory 1008 with data 1007 and a plurality of instructions 1009 stored therein, an induction assembly 1010, a display 1012, and a radiofrequency transceiver 1014 each in communication with a processor 1006.

The memory 1008 may comprise a non-volatile memory and/or a volatile memory. The data 1007 may comprise any type of data including medical data such as: imaging data, e.g., imaging data files, assessment results, diagnoses, measurements, treatment plans, medication schedules, test results, appointment schedules, progress reports, a patient history, etc. The plurality of instructions 1009 may comprise instructions which, when executed by the processor 1006, cause the processor to perform one or more of the techniques described herein. For example, the plurality of instructions 1009 may comprise one or more of heuristic algorithms, machine learning algorithms, device positioning algorithms, algorithms for processing imaging data, algorithms for segregating relevant imaging data from irrelevant imaging data, etc.

The processor 1006 may operate the induction assembly 1010, which may be miniaturized, and which may comprise an induction coil array in some instances, to obtain measurement data and may receive such measurement data from the induction assembly 1010. The processor 1006 may generate numerical or graphical images based on the received measurement data and may output such images to the display 1012. The processor 1006 may also receive user input via the display 1012, or may communicate information to the user via the display 1012. For example, the display 1012 may indicate to the user whether the handheld medical scanning device 1002 is correctly positioned or correctly aligned with respect to the implantable filter, or may indicate to the user whether or not a captured embolus has been detected in the implantable filter.

The processor 1006 may operate the radiofrequency transceiver 1014 to communicate with a remote medical processing system, e.g., a hospital record system or other hospital system. The radiofrequency transceiver 1014 may be configured to communicate over an Institute of Electrical and Electronics Engineers (IEEE) 802.11 (WiFi) link, a Bluetooth link, a Zigbee link, an ultra-wideband (UWB) link, or over any combination thereof. In some cases, the handheld medical scanning device 1002 may comprise multiple radiofrequency transceivers 1014, and different radiofrequency transceivers 1014 may be configured to communicate over different links. In that regard, different radiofrequency transceivers 1014 may be configured to communicate over different frequencies, different time slots, etc.

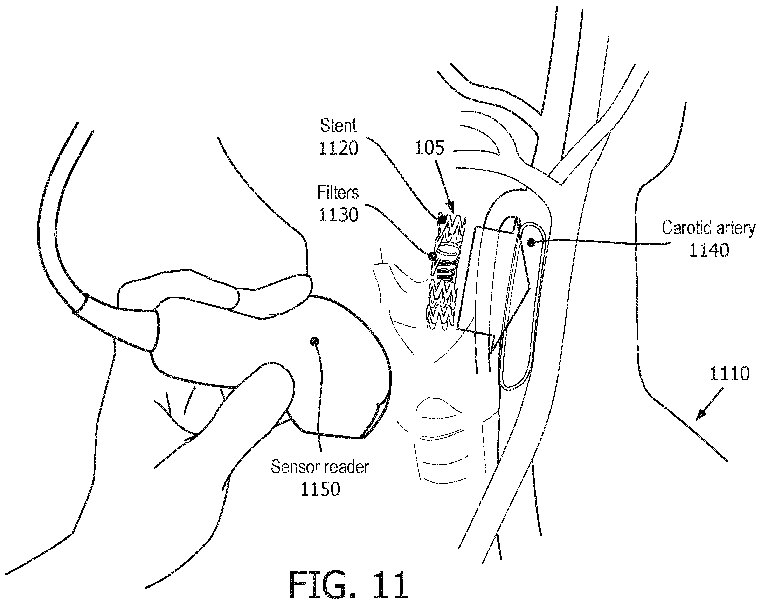

**Figure 11** is a diagrammatic, perspective view of a user 1110 applying an external measurement device, inductance probe, or reader 1150 to the site of the implant 105 in the carotid artery 1140 to perform a self inductance test, in accordance with at least one embodiment of the present disclosure. The implant 105 may include a frame, anchor, or stent 1120 and one or more filters 1130. The arrow shows the location of the implant in the carotid artery.

A key feature of the present disclosure is that when an embolus has been captured, it easily shows up on an inductance test. To achieve this, both the implant 105 and the external measurement device 1150 can be designed or customized with the specific intent of performing this detection in a home setting, with minimal training. Thus, although the external measurement device, inductance probe, or reader 1150 may be an off-the-shelf instrument or may include off-the-shelf components, it may be customized (through any combination of software, firmware, or hardware) such that it primarily or exclusively detects the presence or absence of a captured thrombus within the filters 1130 of the implant 105. For example, the external measurement device 1150 may provide a first indication (e.g., an auditory tone) indicating that it does not currently detect the implant 105, and a second indication (e.g., a different audio tone) when the position and orientation of the external measurement device 1150 are such that the implant 105 can be detected. Then, based on the inductance signal, the external measurement device 1150 can provide a third indication (e.g., a green light) that a captured embolus has not been detected within the implant 105, or a fourth indication (e.g., a red light) that a captured embolus has been detected within the implant 105. The indications may for example be through a user interface providing any combination of visual, auditory, and/or haptic feedback to the user. In some instances, the user interface may be in the external measurement device 1150 itself. In other instances, the user interface may be at least partially within a user device such as a mobile phone, tablet, notebook, laptop, or desktop computer. Thus, the external measurement device 1150 provides a fast and easy test to ascertain if any filter 1130 of the implant 105 has captured any emboli.

In an example, once per x days (for example every day, every 3 days, every 7 days, etc., depending on the patient's perceived risk level), the patient places the external measurement device 1150 over their neck to check if the filter has captured an embolus.

An algorithm in the sensor device could analyze the collected inductance data to assess if an embolus is present and alert the patient. Alternatively, the collected inductance data could be automatically transmitted to a healthcare provider for further analysis. The external measurement device 1150 may include a transceiver that connects to the patient's personal smartphone, and an app that patients install on their personal smartphone for analyzing the collected inductance data.

In an example, the patient is trained to know where to place the external measurement device 1150 over the implant in the carotid artery. The patient places the probe end of the measurement device 1150 on the spot they've been trained to place it. In some cases, the spot may be marked with a tattoo or other marking. The patient then performs a 'sweep', by changing the angle of the external measurement device 1150. The external measurement device 1150 then detects whether an embolus has been captured in the implant 105. The external measurement device 1150 alerts the patient to seek medical attention if a clot or other embolus in the filter has been detected.

It is noted that the external measurement device 1050 may employ one or more sensing techniques that include the emission and/or detection of magnetic fields at any frequency. Examples include, but are not limited to, low-frequency inductance, highfrequency inductance, ECT, ECA, or otherwise, with or without an imaging capability, that is capable of discerning whether an embolus has been captured by the filter.

An algorithm in the sensor device could analyze the collected data to assess if an embolus is present and alert the patient. Alternatively, the collected data could be automatically transmitted to a healthcare provider for further analysis.

If the external measurement device 1050 indicates that the filter did capture an embolus, the patient may go to a hospital for supervision, and may for example be given anti-thrombolytics to dissolve the embolus. If anti-thrombolytics are unsuccessful, the implant 105 device can be removed and replaced with a new implant 105, in a minimally invasive procedure.

**Figure 12** is a diagrammatic, perspective view of a user 1110 wearing a wearable external measurement device, inductance probe, or reader 1250 over the site of the implant 105 in the carotid artery 1140 to perform a self inductance test, in accordance with at least one embodiment of the present disclosure. The implant 105 includes a stent, frame, or anchor 1220 that includes at least one filter 1210. The wearable external measurement device 1250 may for example take the form of a neck wrap that incorporates an array 1260 of inductance coils 1270. The eddy current array (ECA) 1260 integrated into the neck wrap has a method for performing the eddy current test in the carotid. This may be a mechanical mechanism to move the transducer assembly, either manually by the patient or through a motorized actuation.

The ECA 1260 includes a number of coils 1270 arranged in specific pattern called a topology, that generates a sensitivity profile suited to detecting whether or not the implant 105 has captured an embolus. Data acquisition is achieved by multiplexing the coils 1270 in a defined pattern to avoid mutual inductance between the individual coils. The ECA 1260 allows the external measurement device 1250 to conform to the curvature of the neck, while still accurately detecting whether the implant 105 has captured an embolus.

In some embodiments, the wearable external measurement device 1250 may include external markings to help the user align the coils 1270 with the implant 105. In other embodiments, the wearable external measurement device 1250 includes inductance coils 1270 over a large area, and activates the coils 1270 that are closest to the implant 105 to perform the detection of the implant 105 and/or embolus.

In an example, the inductance coils 1270 integrated into the neck wrap include a method for performing a sweep of the inductance plane along the carotid. This may be a mechanical mechanism to move the coil array, either manually by the patient or through a motorized actuation. Alternatively, the array 1260 may be a stationary 2D inductance array that allows for electronically sweeping the imaging plane through a volume of interest. Operation of the wearable external measurement device 1250 may otherwise be similar to operation of the external measurement device 1150 of Figure 11.

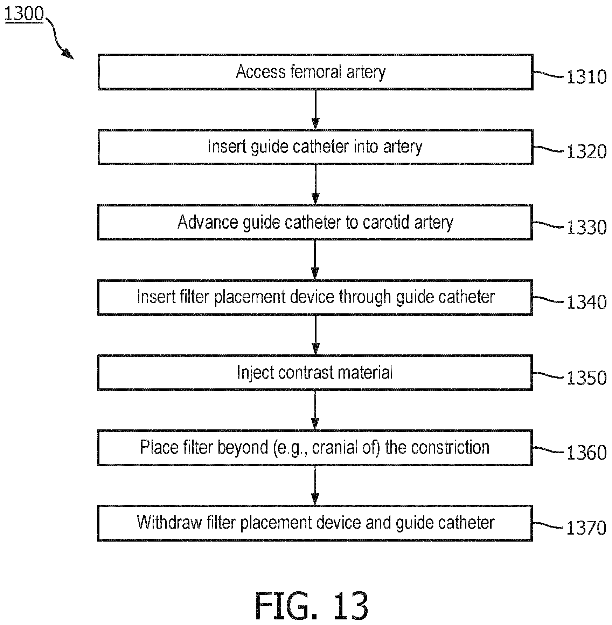

**Figure 13** is a flow diagram showing the steps of an example implantable permanent blood filter placement method 1300, in accordance with at least one embodiment of the present disclosure. For ease of adoption, the method 1300 may be similar to those currently used for implanting a temporary filter in the carotid artery.

In step 1310, the clinician surgically accesses the femoral artery.

In step 1320, the clinician places a sheath or guide catheter into the femoral artery.

In step 1330 the clinician advances the guide catheter to the carotid artery.

In step 1340, the clinician inserts a filter placement device (e.g., a filter placement guidewire) through the guide catheter, and advances it into the carotid artery. In some cases, A catheter with a balloon tip may then be threaded through the guide catheter to a narrowing in the carotid artery (e.g., under X-ray guidance).

In step 1350, the clinician injects contrast material into the carotid artery through the guide catheter and/or balloon catheter, in order to locate a constriction in the carotid artery. In many cases, the most suitable landing site for a permanent filter is downstream of (e.g. cranial of) the constriction. However, even in cases where no constriction is present, the contrast agent may enable the clinician to select a suitable landing site for the filter.

In step 1360, the filter is placed in the artery. The filter, also called an embolic protection device, may for example be inserted cranial of a construction to catch any debris that may break off from the narrowed area of artery during a stenting, balloon angioplasty, or ablation procedure.

In step 1370, the clinician withdraws the filter placement device, guide catheter, and any other intravascular instruments from the patient, and closes access to the femoral artery. The method is now complete.

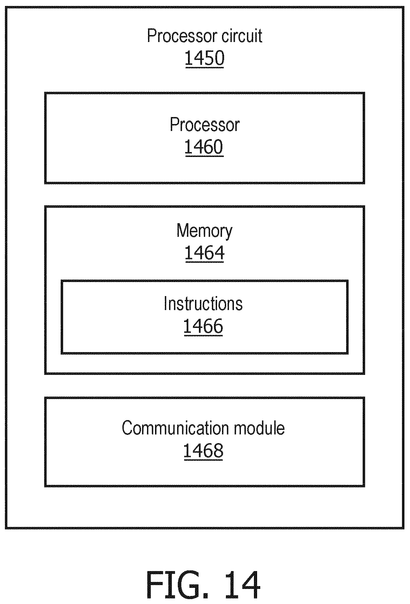

**Figure 14** is a schematic diagram of a processor circuit 1450, according to embodiments of the present disclosure. The processor circuit 1450 may be implemented in the external measurement device 1150 or 1250, or other devices or workstations (e.g., thirdparty workstations, network routers, etc.), or on a cloud processor or other remote processing unit, as necessary to implement the method. As shown, the processor circuit 1450 may include a processor 1460, a memory 1464, and a communication module 1468. These elements may be in direct or indirect communication with each other, for example via one or more buses.

The processor 1460 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1460 may also comprise another hardware device, a firmware device, or any combination thereof configured to perform the operations described herein. The processor 1460 may also be implemented as a combination of computing devices, e.g., a combination of a DSP and a microprocessor, a plurality of microprocessors, one or more microprocessors in conjunction with a DSP core, or any other such configuration.

The memory 1464 may include a cache memory (e.g., a cache memory of the processor 1460), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1464 includes a non-transitory computer-readable medium. The memory 1464 may store instructions 1466. The instructions 1466 may include instructions that, when executed by the processor 1460, cause the processor 1460 to perform the operations described herein. Instructions 1466 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1468 can include any electronic circuitry and/or logic circuitry to facilitate direct or indirect communication of data between the processor circuit 1450, and other processors or devices. In that regard, the communication module 1468 can be an input/output (I/O) device. In some instances, the communication module 1468 facilitates direct or indirect communication between various elements of the processor circuit 1450 and/or the external measurement device 1050 or 1150. The communication module 1468 may communicate within the processor circuit 1450 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the external measurement device) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles) , 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

As will be readily appreciated by those having ordinary skill in the art after becoming familiar with the teachings herein, the implantable blood filter system advantageously provides not only a filter designed explicitly for permanent or long-term implantation in a blood vessel, but also devices and methods for detecting an embolus that has been captured by the filter, in a home setting, with minimal training and effort.

A number of variations are possible on the examples and embodiments described above. For example, the implantable blood filter system can be employed in veins as well as arteries, and in peripheral vasculature as well as cardiac or pulmonary vasculature (e.g., the vena cava), or the vasculature of other body organs including but not limited to the liver, lungs, kidneys, and brain. The implantable blood filter system may be readily detectable through physical inspection and/or inspection of promotional materials. The word "embolus" should be interpreted as including any blood clot, gas bubble, thrombus, dislodged tissue (e.g., fat, plaque, scar tissue, webbing, etc.), or other blockage-causing material large enough to present a risk of ischemic injury to the patient. The term "inductance" should be interpreted as including any sensing method that includes emission and detection of magnetic fields, with or without an imaging capability, that is capable of discerning whether an embolus has been captured by the filter.

The implantable blood filter system can be used for stroke prevention, stenting procedures, and any procedure where a filter (e.g., a temporary filter) is currently placed in the venous or arterial system. The technology described herein may be sized, shaped, or composed differently than shown herein, without departing from the spirit of the present disclosure.

The logical operations making up the embodiments of the technology described herein are referred to variously as operations, steps, objects, elements, components, or modules. It should be understood that these may occur, or be performed or arranged, in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the implantable blood filter system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the implantable blood filter system as defined in the claims. Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

## Claims

1. An implantable blood filter system, comprising:
an anchor fixedly implantable in a blood vessel of a patient;
a metallic induction coil fixedly attached to the anchor;
a filter fixedly attached to the induction coil or the anchor; and
an external inductance measurement device,
wherein the filter comprises a plurality of gaps having a gap size,
wherein the gap size is selected such that:
blood cells can pass through the plurality of gaps; and
an embolus in contact with the filter cannot pass through the plurality of gaps and the embolus is captured by the filter, and
wherein the external inductance measurement device is configured to detect whether the embolus has been captured by the filter.

2. The system of claim 1, wherein, to detect whether the embolus has been captured by the filter, the external inductance measurement device is configured to detect an inductance signature of the embolus.

3. The system of claim 1,
wherein an inductance signature of the filter is configured to change in response to the embolus being captured by the filter, and
wherein, to detect whether the embolus has been captured by the filter, the external inductance measurement device is configured to detect the changed inductance signature.

4. The system of claim 1, wherein the anchor includes the induction coil.

5. The system of claim 1, wherein the anchor includes the filter.

6. The system of claim 1, wherein the induction coil includes the filter.

7. The system of claim 1, wherein the filter comprises a plurality of filter stages.

8. The system of claim 1, wherein the external inductance measurement device comprises a handheld device or a wearable device.

9. A permanently implantable blood filter, comprising:
a stent fixedly implantable in a blood vessel of a patient;
an induction coil fixedly attached to the stent; and
a filter fixedly attached to the stent or the induction coil,
wherein the filter comprises a coil or mesh,
wherein the coil or mesh comprises a plurality of gaps having a gap size,
wherein the gap size is selected such that:
blood cells can pass through the plurality of gaps; and
an embolus in contact with the filter cannot pass through the plurality of gaps and the embolus is captured by the filter.

10. The permanently implantable blood filter of claim 9, wherein at least one of the stent or the coil or mesh comprises a metal.

11. A measurement device, comprising:
at least one induction coil;
a user interface configured to provide visual, auditory, or haptic feedback to a user;
a processor in communication with the user interface and the at least one induction coil; and
a memory operatively coupled to the processor and comprising instructions that direct the processor to:
with at least the at least one induction coil, detect whether an embolus is present in a filter device implanted within the user; and
with the user interface, indicate to the user whether the embolus is present in the filter device.

12. The measurement device of claim 11, wherein the instructions further direct the processor to:
with the user interface, indicate whether the measurement device is properly placed or oriented to detect the embolus in the filter device.

13. The measurement device of claim 11, wherein the instructions further direct the processor to:
if the processor does not detect the filter device, provide, with the user interface, a first indication; or
if the processor detects the filter device, provide, with the user interface, a second indication.

14. The measurement device of claim 13, wherein the instructions further direct the processor to:
if the processor detects that the embolus is not present in the filter device, provide, with the user interface, a third indication; or
if the processor detects that the embolus is present in the filter device, provide, with the user interface, a fourth indication.

15. The system of any of the claims 1 to 8, wherein the permanently implantable blood filter is according to claim 9 or 10 and wherein the external inductance measurement device is according to any of the claims 11 to 14.
